# EUROPÄISCHE PATENTANMELDUNG

(11) **EP 4 413 988 A1**
(43) Veröffentlichungstag der Anmeldung: **14.08.2024**
(21) Anmeldenummer: 23155670.5
(22) Anmeldetag: 08.02.2023
(51) Int. Cl.: A61K 35/57, A61K 38/39, A61K 47/06, A61P 43/00, A61Q 17/00, A61K 8/00, A61K 9/14, A23K 10/00

(54) **VERFAHREN ZUR HERSTELLUNG EINES KERATINPULVERS AUS FEDERN TIERISCHEN URSPRUNGS, KOLLOIDALE LÖSUNG UND KERATINPULVER FÜR DIE TOPISCHE APPLIKATION ENTHALTEND KERATINPARTIKEL UND VERWENDUNG DER KOLLOIDALEN LÖSUNG UND DES KERATINPULVERS**

(71) Anmelder: Rigi Therapeutics AG, 6045 Meggen (CH)
(72) Erfinder:
(74) Vertreter: Pfenning, Meinig & Partner mbB

(57) **Zusammenfassung**

Die vorliegende Erfindung betrifft ein Verfahren zur Herstellung von Keratinpulver des Proteins beta-Keratin aus Federn tierischen Ursprungs mittels Extraktion von Keratin, Filtration der Extraktionslösung unter Erhalt einer kolloidalen Lösung und deren Trocknung zu Keratinpulver. Ebenso betrifft die Erfindung eine kolloidale Lösung für die topische Applikation enthaltend Keratinpartikel des Proteins beta-Keratin und/oder deren Agglomerate oder ein diese Keratinpartikel enthaltendes Keratinpulver. Die kolloidale Lösung und das Keratikpulver finden Verwendung für die Herstellung von Zubereitungen mit therapeutischer, diagnostischer, präventiver oder kosmetischer Zweckbestimmung am Menschen und/oder Tier.

## Beschreibung

Die vorliegende Erfindung betrifft ein Verfahren zur Herstellung von Keratinpulver des Proteins beta-Keratin aus Federn tierischen Ursprungs mittels Extraktion von Keratin, Filtration der Extraktionslösung unter Erhalt einer kolloidalen Lösung und deren Trocknung zu Keratinpulver. Ebenso betrifft die Erfindung eine kolloidale Lösung für die topische Applikation enthaltend Keratinpartikel des Proteins beta-Keratin und/oder deren Agglomerate oder ein diese Keratinpartikel enthaltendes Keratinpulver. Die kolloidale Lösung und das Keratikpulver finden Verwendung für die Herstellung von Zubereitungen mit therapeutischer, diagnostischer, präventiver oder kosmetischer Zweckbestimmung am Menschen und/oder Tier.

Das Stratum corneum (SC) der Epidermis besteht aus mehreren Schichten von keratinisierten Korneozyten, die vom "cornified envelope" ummantelt und durch korneodesmosomale Strukturen verbunden in eine komplexe Lipidmatrix aus flüssigkristallinen, lamellaren Strukturen eingebettet sind.

Die keratinozytäre Lipidsynthese ist weitgehend autonom reguliert und stellt neben Cholesterol und Cholesterolderivaten auch freie Fettsäuren unterschiedlicher Kettenlänge sowie Triglyceride bereit. Zudem werden im endoplasmatischen Retikulum der Keratinozyten Ceramide synthetisiert, die sich substanziell von Molekülen anderer Lipidklassen durch ihre anisomerische Molekülstruktur unterscheiden. Auf Grund von Ladungsunterschieden innerhalb des langkettigen Moleküls können Ceramide spontan lyotrope Mesophasen, also flüssigkristalline Membranstrukturen bilden. Die funktionell bedeutenden Ceramide des Stratum corneum besitzen im Unterschied zu Phospholipiden zwei Alkylketten, die wiederum in der Länge variieren. Zudem weisen diese in Abhängigkeit vom Hydratationsgrad differente Konfigurationen auf, so dass verschiedene Membranmodelle ein komplexes Netzwerk aus Membranabschnitten mit polymorphem Phasenverhalten beschreiben. Die dynamische Ordnung des Gesamtsystems wird in seiner Komplexität zunehmend verstanden und gewinnt mehr und mehr praktische Bedeutung in der Entwicklung moderner galenischer Vehikelsysteme.

Die Barrierefunktion wird zudem wesentlich durch die Quantität der hydrophilen Phase in Mikromilieus bestimmt. Die Verteilung des Wassers innerhalb der Mikrokompartimente des Stratum corneum lässt sich in mindestens zwei Fraktionen beschreiben. Neben einer freien Wasserphase wird eine Fraktion von gebundenem Wasser definiert. Letztere setzt sich aus einer mobilisierbaren und einer fixierten Subfraktion zusammen. Die Nomenklatur zielt dabei auf den dynamischen Austausch hydrophiler Valenzen zwischen den einzelnen Kompartimenten ab. Unter fixiertem Wasser versteht man vordergründig den Wasseranteil, der in Korneozyten durch starke hygroskopische Kräfte, vermittelt durch proteolytisch generierte Aminosäuren, gebunden ist und quasi für den Austausch nur sehr retardiert zur Verfügung steht. Unter besonderen Bedingungen kann durch schwellfähige Membrananteile gebundenes Wasser liberiert und in die freie Wasserphase überführt werden. Hier wird die funktionell bedeutsame Wasserphase des Stratum corneum gesehen. Das freie Wasser wird zudem durch hygroskopische Moleküle, die zusammen als "natürlicher Feuchthaltefaktor (NMF)" bezeichnet werden, in den einzelnen Mikrokompartimenten gebunden und steht im Austausch mit der Wasserphase der vitalen Epidermis sowie der Umwelt (transepidermaler Wasserfluss). Wesentlicher Bestandteil des NMF sind neben Aminosäuren insbesondere Pyrrolidoncarbonsäuren, Lactat, Harnstoff und anorganische Ionen. Diese werden durch die Keratinozyten synthetisiert und in Abhängigkeit vom Differenzierungsgrad freigesetzt.

Im Rahmen der Desquamation werden im Stratum disjunctum, der obersten Schicht des Stratum corneum, Korneodesmosomen und Proteine des cornified envelopes durch Proteolyse abgebaut. Das prädominante Keratin der Korneozyten ist davon aber direkt nicht betroffen. Deshalb bleibt die Integrität der Korneozyten während der Desquamation sehr lange erhalten. Eine wesentliche Funktion der Korneozyten besteht darin Wasser über Diffusion des cornified envelopes für die Membranbildung im interkorneozytären Raum bereitzustellen. Unter therapeutischen Bedingungen wird die Interaktion von Keratin mit epikutan applizierten Wirkstoffen als unbedeutend angesehen, da eine transzelluläre Passage in relevantem Umfang bisher für keinen Wirkstoff nachgewiesen werden konnte. Als Ursache hierfür wird der cornified envelope gesehen, der als Hüll- und Barrieremembran den Korneozyten umgibt. Dennoch beeinflusst der Korneozyt indirekt über seine zentrale Stellung als Wasserreservoir des Stratum corneum wesentlich die Diffusionsbedingungen (Diffusionskoeffizienten) des Stratum corneum. Die kutane Bioverfügbarkeit epikutan applizierter Wirksubstanzen wird nämlich vorwiegend durch die Interaktion des galenischen Systems mit dem Stratum corneum als unmittelbare Kontaktschicht bestimmt. Aus pharmakologischer Sicht sind zwei Prozesse innerhalb des Stratum corneum für den Substanzfluss von Bedeutung, die Barriere- und die Reservoirfunktion. Bedeutsam ist dabei auch, dass das Stratum corneum keine homogene Struktur darstellt, sondern nach der Bildung und kompakter Strukturierung im Stratum compactum durch einen enzymatisch gesteuerten Desquamationsprozess aufgelockert wird (Stratum disjunctum). Somit wird den beiden genannten Funktionsbereichen eine diametrale Bedeutung innerhalb der Strukturebenen des Stratum corneums zuerkannt. Das Maximum der Barrierefunktion liegt demnach im Stratum compactum und dass der Reservoirfunktion im Stratum disjunctum. Letztere dient als privilegierter Akzeptor für epikutan applizierte und liberierte Phasen. Für die kutane Gesamtkinetik eines Wirkstoffs ist dies entscheidend, da Maximalkonzentrationen in tieferen Hautschichten reduziert werden und der Penetrationsprozess verzögert wird. Gleichzeitig können große Mengen liberierter Wirkstoffanteile auch nach relativ kurzen Applikationszeiten aufgenommen und bioverfügbar werden. Insgesamt wird dadurch deutlich, dass die mikromorphologischen Verhältnisse, sowohl als anatomische Varianz (z.B. Felder- vs. Leistenhaut), als auch unter pathologischen Bedingungen (z.B. epidermale Proliferations- bzw. Differenzierungsstörung) unmittelbare Auswirkungen auf das pharmakokinetische Profil einer epikutan applizierten Substanz haben.

Dadurch wird deutlich, dass eine gezielte Beeinflussung des Wassergehaltes des Stratum corneum einen direkten Einfluss auf die physikochemischen Barrierefunktion und damit auf die Pharmakokinetik epikutan applizierter Wirkstoffe hat.

Der Einsatz von Keratin für die medizinische bzw. kosmetische Indikation ist aus folgenden Druckschriften bekannt.

Bislang verfügbares Keratin wird aufgrund von mikrobieller, säure-oder basenbedingter Hydrolyse in kleinere Peptidstrukturen abgebaut und extrahiert und liegt nicht mehr als intaktes und vollständiges Protein Keratin vor (Shavandi, A. et al., Biomater. Sci. 2017, 5, 1699-1735; Gupta, A. et al., J. Chem. Chem. Eng. 2012, 6, 732-737).

Diese Keratin-Peptide sind in ihren Sequenzen kurzkettig und weisen andere Eigenschaften auf (z.B. Molekülgröße, Quellverhalten) als das native, hoch-sequente Keratin-Protein, welches hier erstmalig gewonnen werden konnte. Im Unterschied zu bisherigen Verfahren wurde auf eine Hydrolyse zur Extraktion verzichtet und stattdessen das Protein über das Brechen der Disulfidbrücken für die Extraktion zugänglich gemacht. Dadurch bleiben die Primärstruktur und die Sekundärstruktur des Proteins Keratin als β-Faltblatt erhalten

Ausgehend hiervon war es Aufgabe der vorliegenden Erfindung, kolloidale Lösungen bereitzustellen, die als Grundlage für Zubereitungen dienen, die eine Interaktion sowohl mit hydrophilen wie lipophilen Komponenten bzw. Wirkstoffen zeigen. Zudem sollen diese kolloidalen Lösungen auf einfache Weise herstellbar sein.

Diese Aufgabe wird mit dem Verfahren mit den Merkmalen das Anspruchs 1 und die kolloidale Lösung mit den Merkmalen des Anspruchs 7 gelöst. In Anspruch 18 werden erfindungsgemäße Verwendungen angegeben. Die weiteren abhängigen Ansprüche nennen bevorzugte Ausführungsformen.

Erfindungsgemäß wird ein Verfahren zur Herstellung von Keratinpartikeln aus Federn tierischen Ursprungs, bei dem
a) eine Extraktion des Proteins beta-Keratin in einer eine chemische Denaturierung auslösenden Extraktionslösung enthaltend mindestens ein Denaturierungsagens, mindestens eine Base, mindestens ein Reduktionsmittel und mindestens eine Puffersubstanz erfolgt,
b) die Extraktionslösung aus Schritt a) einer Filtration unterzogen wird, bei der eine kolloidale Lösung der Keratinpartikel des Proteins beta-Keratin erhalten wird,
c) die kolloidale Lösung aus Schritt b) per Gefriertrocknung, Sprühtrocknung, Vakuumtrocknung, Lufttrocknung, Wärmetrocknung, Infrarottrocknung und/oder Mikrowellentrocknung getrocknet wird unter Erhalt eines Keratinpulvers enthaltenden Pulvers.

Die vorliegende Erfindung betrifft somit den Einsatz von Keratinpartikeln des Proteins beta-Keratin, die durch chemische Denaturierung aus Vogelfedern als Protein Keratin extrahiert und in flüssigen oder halbfesten Zubereitungen zur epikutanen Applikation eingesetzt werden.

Bei der Gewinnung von Keratin aus Wollhaaren werden alpha-und beta-Keratin gewonnen, sogenanntes "soft fiber" (alpha) oder "hard fiber"(beta)-Keratin, also zwei unterschiedliche Arten des Keratins. Die hier durchgeführte Extraktion mit Harnstoff (Urea), führt im alkalischen Bereich dazu, dass die native Form des beta-Keratins als gesamtes Protein aus den Federn gewonnen wird und so den hohen Anteil an seiner Sekundärstruktur, dem β-Faltblatt, behält, der die hohe Wasserbindekapazität dieses Keratins bedingt. Beta-Keratin wird insbesondere aus Vogelfedern gewonnen. Es ist reich an den Aminosäuren Glycin und Alanin und besitzt wenig Cystein, Prolin und Hydroxyprolin.

Dabei wird die Interaktion des Keratins mit hydrophilen Substanzen als Reservoir zur gezielten Beeinflussung deren Pharmakokinetik genutzt. Durch die Kombination des Keratins mit Wasser, Aminosäuren, hygroskopischen Substanzen, Peptiden und/oder Proteinen soll zudem eine Substitution der physikalischen Barriere erzielt werden.

Vorzugsweise weist die Extraktionslösung einen pH-Wert von 8 bis 13 auf.

Es ist bevorzugt, dass das mindestens eine Denaturierungsagens ausgewählt ist aus der Gruppe bestehend aus Harnstoff, Thioharnstoff, Guanidinhydrochlorid, Natriumdodecylsulfat (SDS) und Mischungen hiervon.

Es ist weiter bevorzugt, dass die mindestens eine Base ausgewählt ist aus der Gruppe bestehend aus Natriumhydroxid, Kaliumhydroxid und Mischungen hiervon.

Das mindestens ein Reduktionsmittel ist vorzugsweise ausgewählt aus der Gruppe bestehend aus β-Mercaptoethanol, Cysteaminen, Cysteine, Glutathion, Natriumdisulfit, Natriumsulfid, Natriumhydrogensulfit, Natriumdithionit, Natriumthiosulfat, Dithiotreitol (DTT), Thioglykolsäure und ihre Salze, Thioharnstoff, Tricarboxyethylphosphan (TCEP) und andere Phosphane, Ammoniumchlorid und Mischungen hiervon.

Es ist bevorzugt, dass die mindestens eine Puffersubstanz ausgewählt ist aus der Gruppe bestehend aus Tris(hydroxymethyl)aminomethan, Natriumdodecylsulfat (SDS), Tris/Salzsäure (HCl), Ethylendiamintetraessigsäure (EDTA)/Tris, Kaliumchlorid-Natriumhydroxid (KCI-NaOH), Natriumhydrogencarbonat (NaHCO₃), Dithiotreitol (DTT)/Tris und Mischungen hiervon.

Es ist bevorzugt, dass die Extraktionslösung mindestens eine der folgenden chemischen Komponenten enthält:
- mindestens ein Oxidationsmittel ausgewählt aus der Gruppe bestehend aus Wasserstoffperoxid, Kaliumpermanganat, Natriumperborat, Peroxyessigsäure, Perameisensäure und Mischungen hiervon,
- mindestens eine Säure ausgewählt aus der Gruppe bestehend aus Salpetersäure, salpetrige Säure, hypo-und hyperhalogenige Säuren und Mischungen hiervon,
- mindestens ein ionisches Agens ausgewählt aus der Gruppe bestehend aus 1-Butyl-3-methyl-imidazolium (BMIM)-chlorid, 1-Butyl-3-methyl-imidazolium (BMIM)-Bromid, 1-Butyl-3-methyl-imidazolium (BMIM)-tetrafluoroborat, Amidchlorid und Mischungen hiervon,

Eine bevorzugte Ausführungsform des erfindungsgemäßen Verfahrens sieht vor, dass bei der Extraktion in Schritt a) mindestens einer der folgenden Schritte erfolgt:
- eine mechanische Zerkleinerung, insbesondere durch Zermahlen via Ultraschall, bevorzugt im Frequenzbereich von 20 bis 50 Hertz, mit einer Kugelmühle und/oder mit einer Schneidmühle, bevorzugt Ultrathurax,
- eine thermische Denaturierung, insbesondere bei Temperaturen von 70°C bis 150°C, und/oder elektrochemische Denaturierung,
- eine Fällung der Extraktionslösung aus Schritt a), insbesondere ausgelöst durch eine pH-Änderung, Zugabe eines Co-Solvens und/oder eines Salzes,
- eine mikrobielle und enzymatische Extraktion über
   - gram negative Bakterien ausgewählt aus der Gruppe bestehend aus *Stenotrophomonas* sp., *Chrysebacterium* sp., *Vibrio* sp. und Mischungen hiervon,
   - gram positive Bakterien ausgewählt aus der Gruppe bestehend aus *Bacillus sp., Kocuria rosea* und Mischungen hiervon
   - saprophytische und/oder parasitische Pilze und/oder
   - Mischungen hiervon,
- Behandlung mit Mikrowellenstrahlung, insbesondere Mikrowellenstrahlung bis zu 960 Watt und 2450 Hertz,
- Einsatz von Stromexplosion und/oder überkritischem Wasser und/oder.
- Kombinationen hiervon.

Es ist bevorzugt, dass das Protein beta-Keratin in seiner Sekundärstruktur als β-Faltblatt vorliegt. Im Unterschied zum alpha- (= soft fiber) Keratin zeigt beta- (= hard fiber) Keratin einen hohen Anteil an eng zusammengedrehter und durch Disulfidbrücken stabilisierter β-Faltblattstrukturen, die eine hohe Stabilität des hard fiber-Keratins gewährleisten.

Vorzugsweise erfolgt die Filtration über Dialyse und/oder Ultrafiltration (Cross-Flow-Filtration).

Die Federn tierischen Ursprungs sind vorzugsweise ausgewählt aus der Gruppe bestehend aus Federn von Hühnern, Gänsen, Puten, Enten, Truthähnen, Fasanen, Straußen, Nandus, Emus, Wachteln und Mischungen hiervon.

Erfindungsgemäß wird ebenso eine kolloidale Lösung für die topische Applikation bereitgestellt, die Keratinpartikel des Proteins beta-Keratin und/oder deren Agglomerate enthält.

Es ist bevorzugt, dass die Keratinpartikel in kolloidaler Lösung eine Partikelgröße im Bereich von 5 nm bis 500 nm, bevorzugt 70 nm bis 350 nm aufweisen.

Es ist bevorzugt, dass zur Stabilisierung die kolloidale Lösung Additiva enthält, die bevorzugt ausgewählt sind aus der Gruppe bestehend aus
- Proteinen z.B. Albumine
- Kohlenhydraten, wie Saccharose, Lactose, Glucose, Fructose, Mannitol, Sorbitol und Süßstoffen wie Saccharin-Natrium, Natriumcyclamat, Aspartam, Stärke und modifizierte Stärke, Cyclodextrine und/oder Mischungen hiervon,
- Polyanionische Tenside, wie Natriumdodecylsulfat, Natriumcetylstearylsulfat, Cetylstearylalkohol (emulgierend), Natriumdioctylsulfosuccinat und/oder Mischungen hiervon,
- Nichtionische Tenside, wie Fettalkohole und Sterole, Sortitanfettsäureester, Polyoxyethylen-Sorbitanfettsäureester, Polyoxyethylen-Fettsäureglyceride, Macrogol-1000-glycerolmonofettsäureester, Polyoxyethylen-Fettsäureester, Polyoxyethylen-Fettalkoholether, Glycerolfettsäureester, Saccharosefettsäureester, Poloxamere und/oder Mischungen hiervon,
- Gelbildner wie z.B. Polyacrylate, Cellulosederivate, wie Methycellulose, Methylhydroxyproylcellulose Hydroxypropylcellulose, Hydroxyethylcelluloseund/oder Ethylcellulose, Carmellose-Natrium und/oder Mischungen hiervon,
- Verdickungsmittel, wie z.B. Tragant, Xantham, arabisches Gummi, Guargalactomannan, Alginate, Bentonit, und/oder Mischungen hiervon,
- Filmbildnern, wie Methacrylsäure-Acrylaten, Polyvidon, Polyvinylalkohol und/oder Mischungen hiervon,
- Polymeren wie Macrogole, Gelatine und/oder Mischungen hiervon.

Erfindungsgemäß werden auch Keratinpulver des Proteins von beta-Keratin für die topische Applikation bereitgestellt, die aus der zuvor beschriebenen kolloidalen Lösung herstellbar ist. Das Keratinpulver ist durch Trocknung der kolloidalen Lösung, insbesondere durch Gefriertrocknung, Sprühtrocknung, Vakuumtrocknung, Lufttrocknung, Wärmetrocknung, Infrarottrocknung und/oder Mikrowellentrocknung.

Es ist bevorzugt, dass die Keratinpartikel im Keratinpulver eine Partikelgröße im Bereich von 1 bis 250 µm, bevorzugt von 5 bis 30 µm aufweisen.

Verwendung findet die zuvor beschriebene kolloidale Lösung sowie das zuvor beschriebene Keratinpulver zur Herstellung von Zubereitungen mit therapeutischer, diagnostischer, präventiver oder kosmetischer Zweckbestimmung an Lebewesen, insbesondere an Menschen und/oder Tieren.

Eine bevorzugte Ausführungsform sieht vor, dass die kolloidale Lösung mindestens einen kleinmolekularen und/oder biotechnologischen Wirkstoff enthält. Vorzugsweise ist der Wirkstoff ausgewählt aus der Gruppe bestehend aus Glucocorticoiden, Calcineurininhibitoren, Januskinase-Inhibitoren, Antibiotika, und/oder selbst ein Protein, Fraktionsprotein, Peptid, Enzym, Antikörper, Antikörperfragment, RNA- und/oder DNA-Molekül, und Mischungen hiervon. Eine weitere bevorzugte Ausführungsform sieht vor, dass ein Molekül enthalten ist, das ein Target für einen dieser Wirkstoffe darstellt.

Es ist ferner bevorzugt, dass die kolloidale Lösung kosmetische Wirk-und Hilfsstoffe enthält, bevorzugt ausgewählt aus der Gruppe bestehend aus Aminosäuren, Harnstoff, Glycerol, Hyaluronsäure, Zuckern- und zuckerähnlichen Derivaten, Extrakten oder Wachsen aus Pflanzen oder von Tieren stammenden Teilen und Mischungen hiervon.

Eine bevorzugte Ausführungsform sieht vor, dass spezifische und/oder unspezifische Verbindungen mit Wirk- und/oder Hilfsstoffen durch kovalente und/oder nicht-kovalente Bindungen, bevorzugt über ausgewählte Gruppen bestehend aus Carbonsäuregruppe, Aminogruppen, Thiolgruppen und/oder Hydroxylgruppen, gebildet werden.

Es ist bevorzugt, dass zur Stabilisierung der kolloidalen Lösung mit/ohne Wirk-und/oder Hilfsstoffe diese als liposomale Systeme in uni- oder multilamellare Vesikel unterschiedlicher oder einheitlicher Größe eingebaut sind durch Zusatz von amphiphilen Molekülen. Diese amphiphilen Moleküle sind bevorzugt ausgewählt aus der Gruppe bestehend aus:
- Phospholipiden, bevorzugt Lecithin, DODAB, DPPC, DSPC, DSTAP und/oder Mischungen hiervon,
- kationischen Lipiden, bevorzugt ALC-0315,
- PEGylierte Lipide, bevorzugt ALC-0159
- Prostaglandine und -modifikationen, bevorzugt PGE1, PGD2, PGE2, 15-keto PGE1 und/oder Mischungen hiervon,
- Ceramiden, bevorzugt ausgewählt aus der Gruppe bestehend aus Ceramiden der Kopfgruppen NS, NH, NP, NDS, AS, AH, ADS, AP der Kettenlängen C₁₀ bis C₂₆ und/oder Mischungen hiervon,
- Ceramiden, bevorzugt ausgewählt aus der Gruppe bestehend aus Ceramiden der Kopfgruppen EOS, EOH, EOP der Kettenlängen C₁₀ bis C₃₂ und/oder Mischungen hiervon,
- Cholesterol, Cholesterolderivaten und/oder Mischungen hiervon,
- Fettsäuren, bevorzugt ausgewählt aus der Gruppe bestehend aus Fettsäuren der Kettenlängen C₁₀ bis C₃₂ und/oder Mischungen hiervon.

Dabei ist es bevorzugt, dass die Applikation an Lebewesen, insbesondere am Menschen, die Haut, Schleimhaut (inkl. Konjunktiven) oder den Hautanhangsgebilden (inkl. Nägeln und Haare) betrifft, insbesondere zur Substitution der Barrierefunktion bzw. von Bestandteilen der epidermalen Barriere.

Die erfindungsgemäßen kolloidalen Lösungen und Keratinpulver werden ebenso zur Herstellung von
- flüssigen Grundlagen, insbesondere Lösungen, Emulsionen, Suspensionen und/oder Kolloiden,
- halbfesten Grundlagen, insbesondere Suspensionssalben, Salben, Cremes, Gele, Pasten, Kolloiden und/oder Suppositorien,
- fester Grundlagen, insbesondere Pulver, Tabletten, Granulate, Pellets, Kapseln und/oder Inserts
verwendet.

Die erfindungsgemäßen kolloidalen Lösungen und Keratinpulver werden ebenso zur Herstellung von
- Nahrungsmitteln und Nahrungsergänzungsmitteln,
- Tierfutter,
- Dünge- und/oder Pflanzenschutzmitteln für Pflanzen und/oder Böden,
- technischen Hilfsstoffen z.B. in/als Filtersystem, Klebstoff- und/oder Haftvermittler, Konsistenzgeber, Füllstoff, Absorber für hydrophile oder lipophile Stoffe, geladene oder ungeladene Stoffe,
- Verpackungsmaterialien und/oder Bedarfsgegenständen,
- Textilien und/oder Fasern mit/ohne Funktion, insbesondere wasserabweisend, atmungsaktiv,
- Medizinprodukten, insbesondere Pflaster, Wundauflagen, Tamponaden, wund- und/oder hautversorgende Kleidung.

Anhand der nachfolgenden Figuren und des Beispiels soll der erfindungsgemäße Gegenstand näher erläutert werden, ohne diesen auf die hier gezeigten spezifischen Ausführungsformen einschränken zu wollen.
- Fig.1: zeigt REM-Aufnahmen von lyophilisiertem Haarkeratin
- Fig.2: zeigt TEM-Aufnahmen von A) Federkeratin (0,5 mg/ml) und B) Haarkeratin (0,5 mg/ml)
- Fig. 3: zeigt fluoreszenzspektroskopische Aufnahmen von 5% (w/w) Keratinpartikel in einer Basiscreme DAC (mit A) Federkeratin. B) und C) Haarkeratin)
- Fig. 4: zeigt Aufnahmen von Penetrationsuntersuchungen von A) Federkeratin und B) Haarkeratin mit jeweils 5% (w/v) in Basiscreme DAC
- Fig. 5: zeigt eine Aufnahme von Penetrationsuntersuchung von Federkeratin 5% (w/v) in Basiscreme DAC mit einer Fluoreszenzaufnahme
- Fig. 6: zeigt Zytotoxizitätsdaten als Index an lyophilisiertem und kolloidalem Federkeratin an Keratinozyten (NHEK) und an lyophilisiertem und kolloidalem Federkeratin an dermalen Fibroblasten (NHDF)
- Fig. 7: zeigt das Ergebnis eines Scratch-Tests mit Darstellung der epithelisierten Fläche von jeweils 3 unabhängigen Versuchen an lyophilisiertem Federkeratin an Keratinozyten (HaCaT)
- Fig. 8: zeigt das Ergebnis eines Scratch-Tests mit Darstellung der epithelisierten Fläche von jeweils 3 unabhängigen Versuchen an kolloidalem Federkeratin an Keratinozyten (HaCaT)
- Fig. 9: zeigt das Ergebnis eines Scratch-Tests mit Darstellung der epithelisierten Fläche von jeweils 3 unabhängigen Versuchen an lyophilisiertem Federkeratin an Keratinozyten (NHDF)
- Fig. 10: zeigt das Ergebnis eines Scratch-Tests mit Darstellung der epithelisierten Fläche von jeweils 3 unabhängigen Versuchen an kolloidalem Federkeratin an Keratinozyten (NHDF)
- Fig. 11: zeigt anhand eines Diagramms die Wasseraufnahme von Federkeratin nach Zugabe von 25 µl T₂O (1 µCi) zu 5 mg lyophilisiertem Keratin, n=3 (Kontaktzeiten: 1 min, 5 min, 1 h, 16 h, 24 h), Zentrifugation, Abnahme des Überstandes, Bestimmung der radioaktiven Dosis
- Fig. 12: zeigt in einer Darstellung die Kultivierung von HaCaT-Zellen mit 1mg Federkeratin
- Fig.13: zeigt die Wasseraufnahme von Harnstoff, Glycerol und Keratin sowie Mischungen von Keratinpartikeln mit Glycerol (50:50) und Keratinpartikeln mit Harnstoff in unterschiedlichen Verhältnissen (z B. 95:5 (orange); 50:50 (hellblau); 5:95 (braun)) im Klimaschrank (Raumtemperatur und 95% relative Luftfeuchtigkeit) über die Zeit
- Fig.14: zeigt die Wasserabgabe von Harnstoff, Glycerol und Keratin sowie Mischungen von Keratinpartikeln mit Glycerol (50:50) und Keratinpartikeln mit Harnstoff in unterschiedlichen Verhältnissen (z B. 95:5 (orange); 50:50 (hellblau); 5:95 (braun)) im Klimaschrank (Raumtemperatur und 50% relative Luftfeuchtigkeit) über die Zeit
- Fig. 15: zeigt die Tertiärstruktur des beta-Keratins aus Vogelfedern

### Beispiel

Es wurde versucht, die Funktionalität eines Korneozyten nachzustellen. Dazu wurde aus verschiedenen natürlichen Keratinquellen Keratin extrahiert und entsprechende Partikel generiert, die zunächst als mechanische Stabilisierung von Lipidmatrices in verschiedenen halbfesten Zubereitungen untersucht wurden. Dabei war beabsichtigt, die Keratinpartikel mit bipolaren Lipiden zu ummanteln, um Keratosomen zu entwickeln.

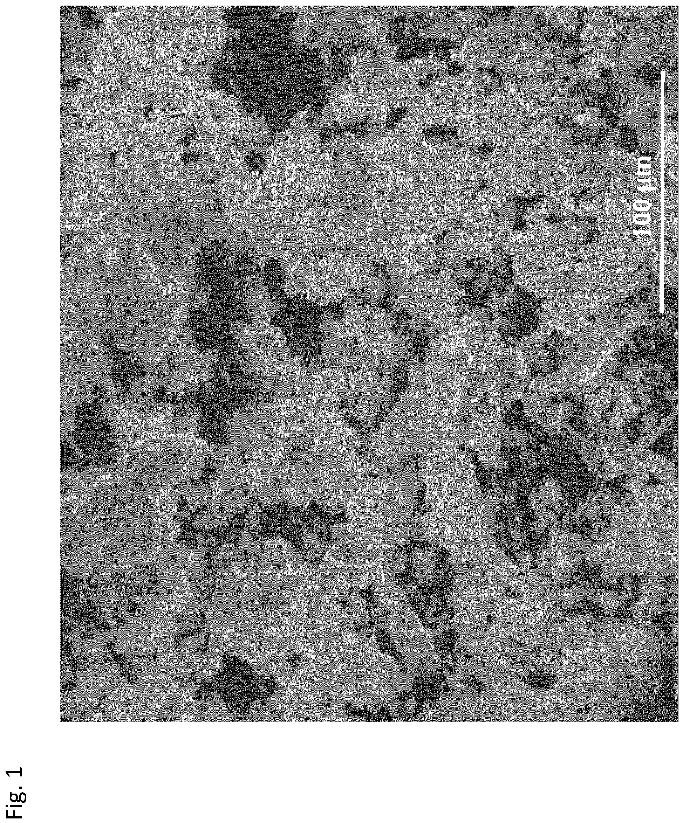

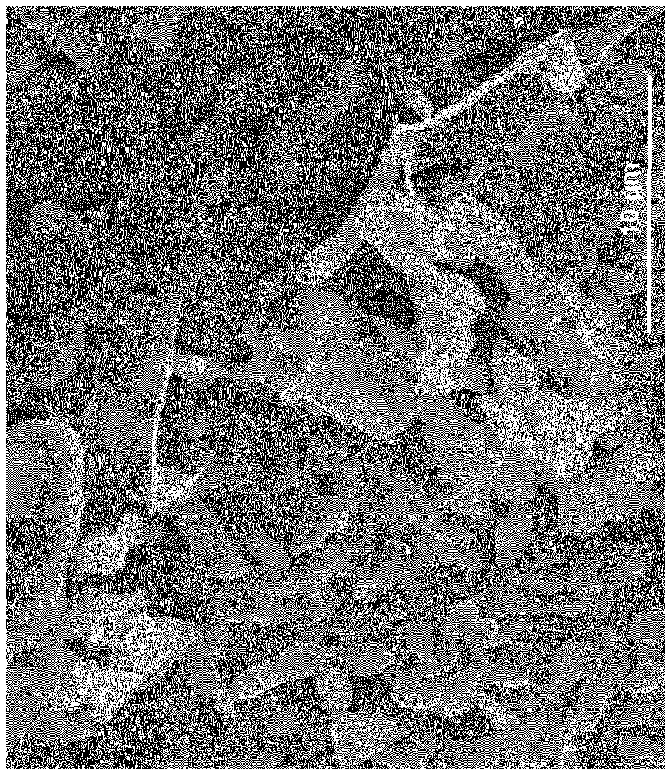

Zunächst wurden verschiedene Extraktionsmethoden an humanem Haar (alpha-Keratin) getestet. Überwiegend wurde die chemische Denaturierung zur Zersetzung des Haares genutzt. Dafür wurden Urea, Thiourea und Guanidiuniumhydrochlorid eingesetzt. Zur Unterstützung der Denaturierung fanden als Reduktionsmittel β-Mercaptoethanol, Cysteamine, L-Cystein Verwendung. Höchste Ausbeuten wurden mit einer Extraktionslösung aus 5 M Guanidiuniumhydrochlorid, 10 % Cysteamine, 25 mM Tris bei pH 8,5 erzielt. Auf Grund der Unbedenklichkeit der Substanzen Urea und L-Cystein wurde zur Isolierung des Keratins eine Extraktionslösung bestehend aus 10 M Urea, 100 mM L-Cysteine, 25 mM Tris-HCl bei pH 10,5 ausgewählt. Nach erfolgreicher Extraktion wurde die Extraktionslösung dialysiert (cutoff: 6-8 kDa, regenerierte Cellulose, SpectraPor^{®}), dabei entsteht eine kolloidale Lösung, bei hoher Proteinkonzentration und großen Dialyseschritten (5-6 I) fällt Keratin im Dialyseschlauch aus. Final wurde das Dialysat lyophilisiert, wodurch ein weißes Pulver entsteht. Dieses Pulver wurde mittels Rasterelektronenmikroskopie (REM) untersucht (Fig. 1). Die Partikel aus Haarkeratin weisen eine variierende Größe und Form auf. Dabei wurde definiert, dass die Partikel in der finalen Formulierung eine Größe von >600 nm aufweisen sollten, um eine Penetration durch die Hornschicht der Haut zu vermeiden.

Aus regulatorischen Gründen wurde als alternative Keratinquelle Federkeratin (beta-Keratin) von verschiedenen Vogelarten verwendet. Hierzu wurden Rohfedern von Hühnern, Gänsen und Enten untersucht. Aus praktischen Erwägungen wurden insbesondere Hühnerfedern für die weiteren Untersuchungen eingesetzt.

Deshalb wurde die Extraktion von Federkeratin an den etablierten Extraktionsprozess für Haarkeratin angepasst. Dabei wurde beobachtet, dass im Vergleich zu allen anderen untersuchten Extraktionsverfahren mit der ausgewählten Extraktionslösung bestehend aus 10 M Urea, 100 mM L-Cystein, 25 mM Tris-HCl bei pH 10,5 die höchste Ausbeute zu erzielen ist. Die optionale Zugabe vom 1 M Ammoniumchlorid, verhindert die Carbamylierung am Protein, und die damit einhergehenden Veränderungen der Proteineigenschaften.

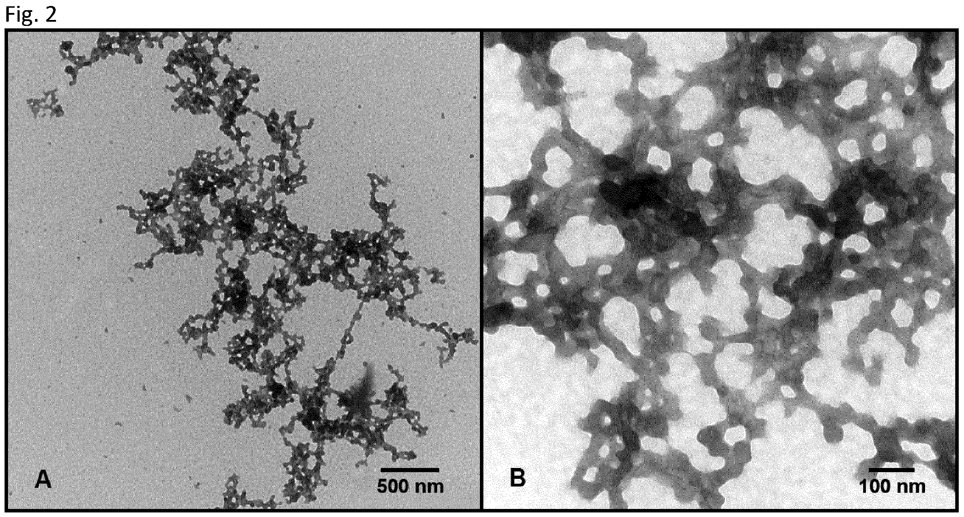

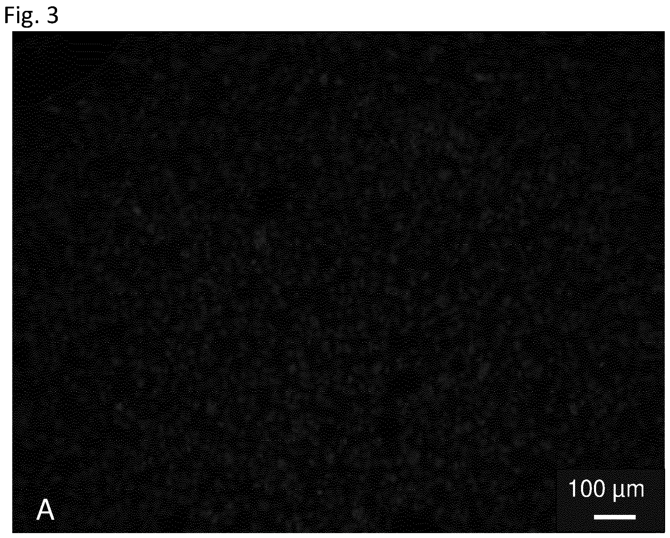

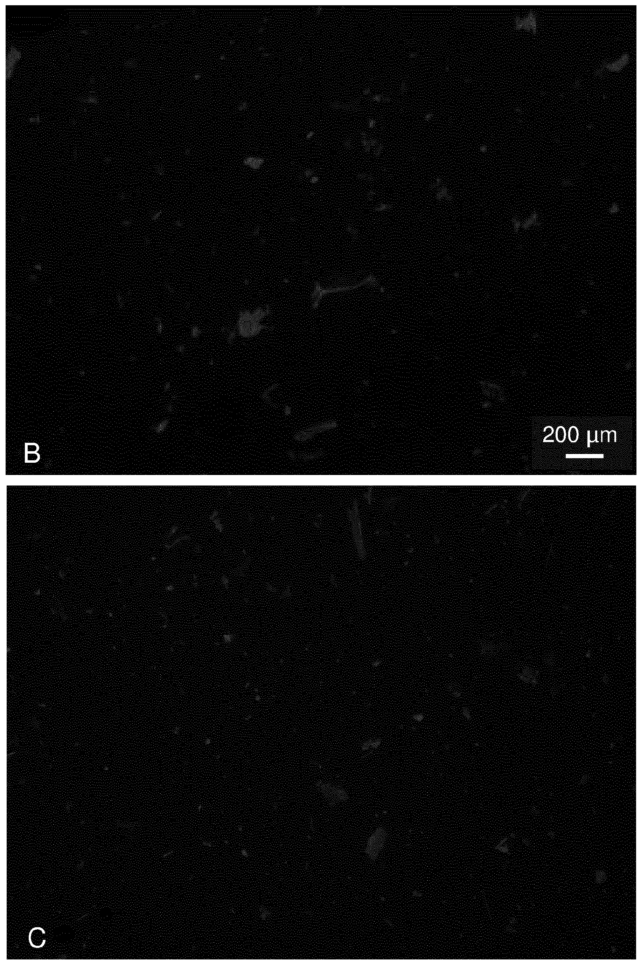

Zur Größenbestimmung wurden die Feder- und Haarkeratinpartikel vergleichend mittels Transmissionselektronenmikroskopie (TEM) im negativ Stain untersucht (Fig. 2). Haarkeratinpartikel in kolloidaler Lösung wiesen eine Größe von ca. 40-75 nm, hingegen für die Federkeratinpartikel eine Größe von ca. 20-35 nm auf. Zur Visualisierung der Keratinpartikel für die Penetrationsuntersuchung wurden diese mit 2-Aminobenzoyl (Abz) fluoreszenz-markiert (Reaktion mit Isatosäureanhydrid unter basischen, denaturierenden Bedingungen). 5 % (w/w) fluoreszenzmarkiertes Haar- und Federkeratin wurde in Basiscreme DAC formuliert und nach Objektträgerausstrich fluoreszenzmikroskopisch untersucht (Fig. 3). Die einzelnen Partikel aus den kolloidalen Lösungen, welche mittels TEM identifiziert wurden, bilden in Basiscreme DAC anteilig Aggregate. Für Federkeratin ist eine homogene Verteilung der zusammengelagerten Partikel zu erkennen, die Partikelgrößen liegen zwischen 20 und 35 µm. Haarkeratin hingegen lagert sich zu größeren, kristallartigen Partikeln zusammen. Die Partikelgrößen liegen hier zwischen 20 und 140 µm.

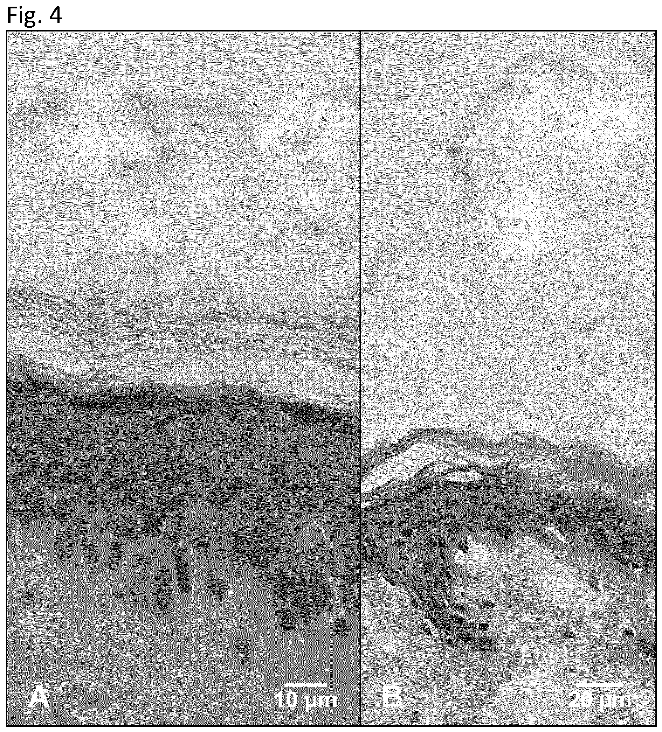

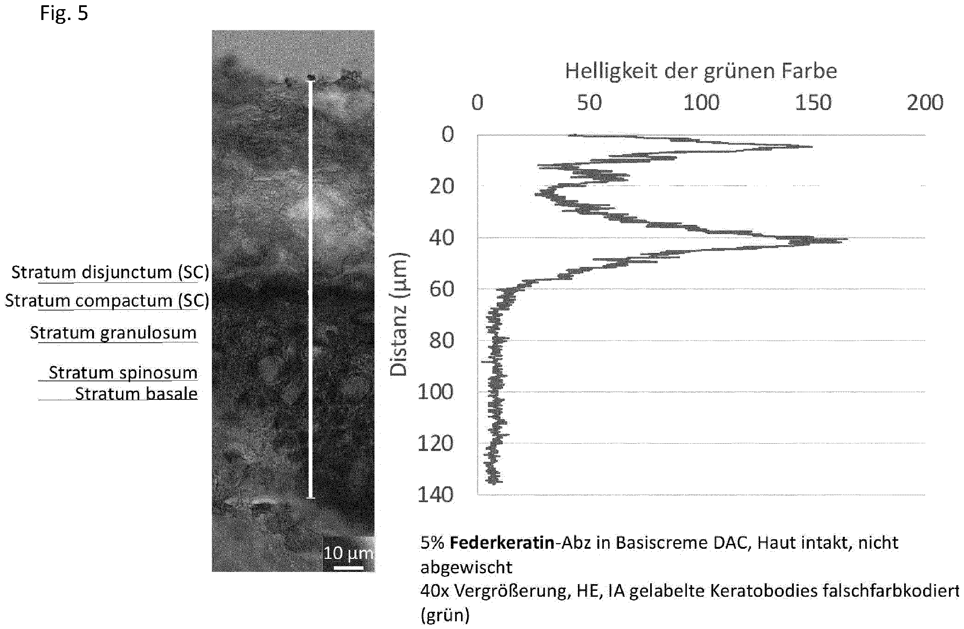

Mittels Penetrationsuntersuchungen von Haar- und Federkeratin (jeweils 5% (w/w)) an *ex vivo* Humanhaut (Franzzelle) in Basiscreme DAC konnte zudem nachgewiesen werden, dass die Keratinpartikel nicht in tiefe Hautschichten diffundieren, sondern in den oberen Anteilen des SC (Stratum disjunctum) verbleiben (Fig. 4 und Fig. 5). Um die Interaktion der Federkeratinpartikel mit den SC-Lipiden bzw. Effekte von Lipidbeschichtungen besser einschätzen zu können, wurde bei verschiedenen pH-Werten das Zeta-Potential bestimmt (Tab. 1).

**Tab. 1: Zeta-Potential von Federkeratin und fluoreszenz-markiertem Federkeratin jeweils 10mg/ml in 10mM Kaliumchlorid bei verschiedenen pH-Werten. Messung durchgeführt von Fabio.**

| **pH-Wert** | **Federkreatin 10 mg/mL** | **Federkreatin-Abz** |
|---|---|---|
| 10,5 | -25 mV | -24,2 mV |
| 7,6 | -1,5 mV | -23,3 mV |
| | Versuch 1: -17,8 mV | |
| 5,5 | -20,2 mV | -23,3 mV |

Die Keratinpartikel weisen eine negative Ladung auf, was sie zur Beschichtung mit positiv-geladenen Lipiden prädestiniert.

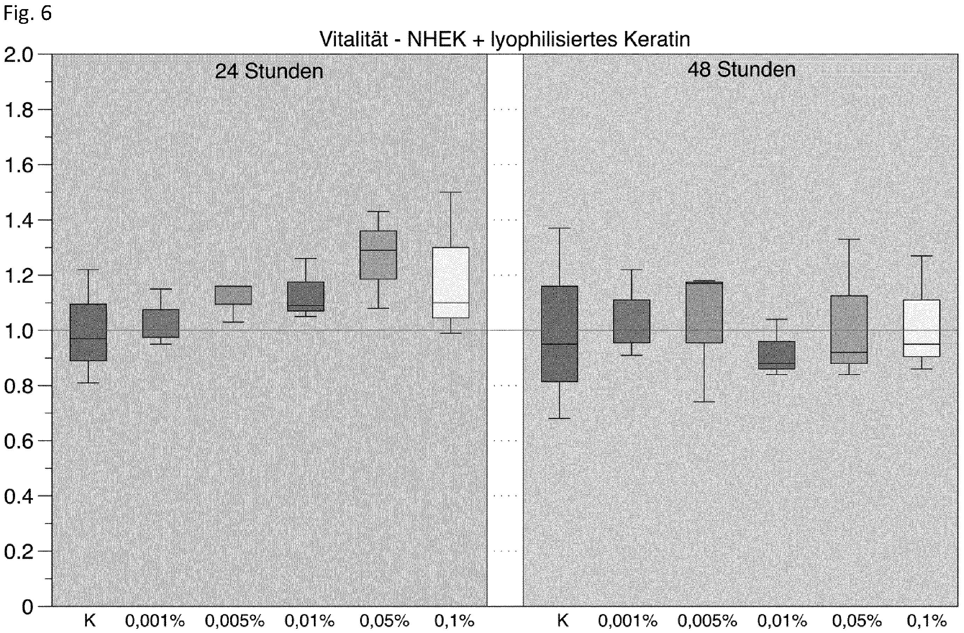

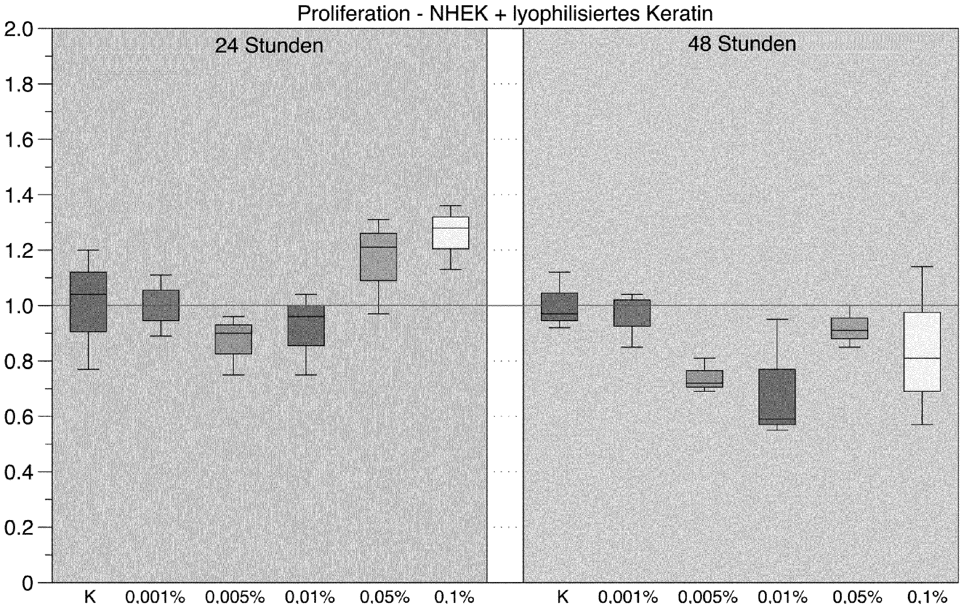

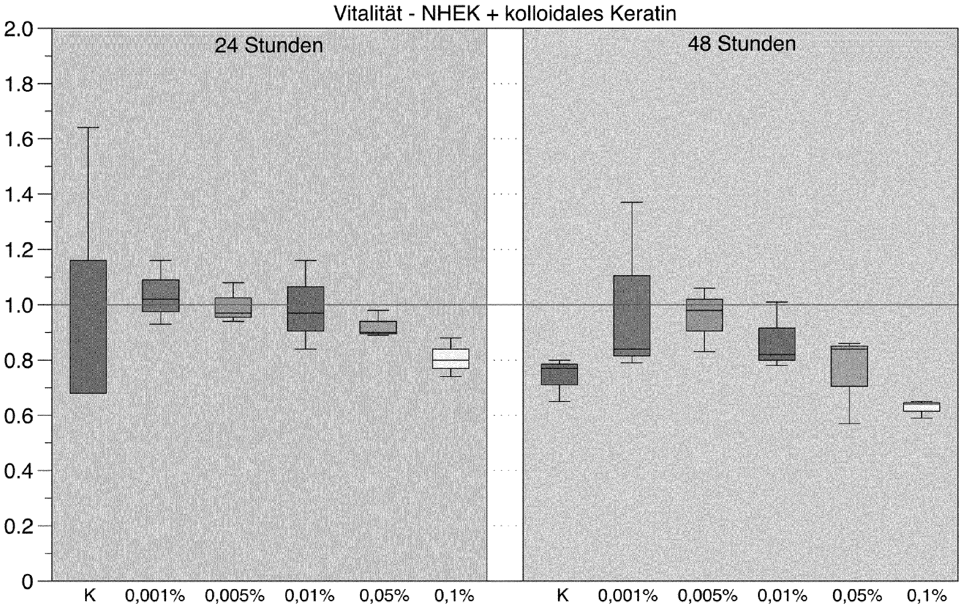

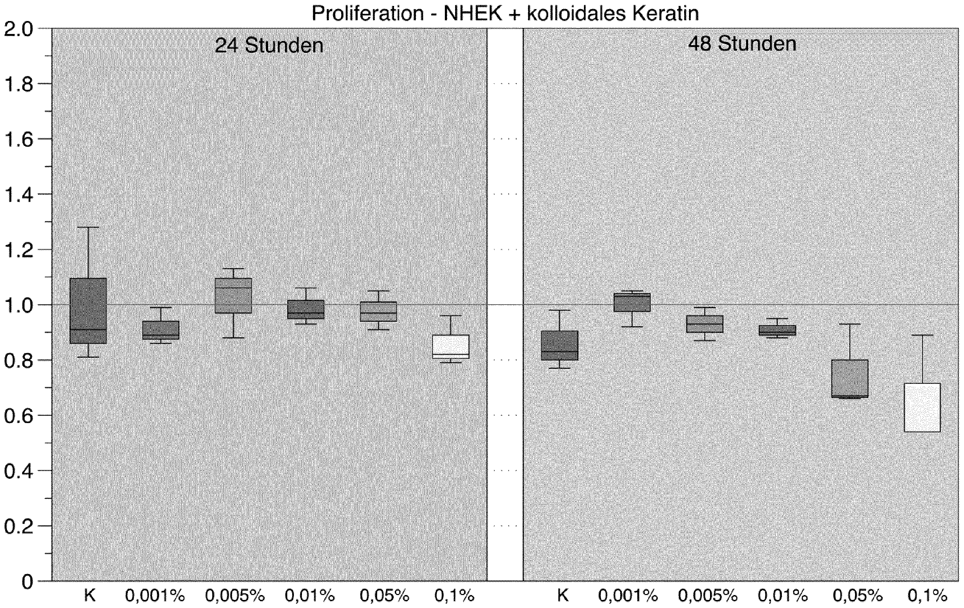

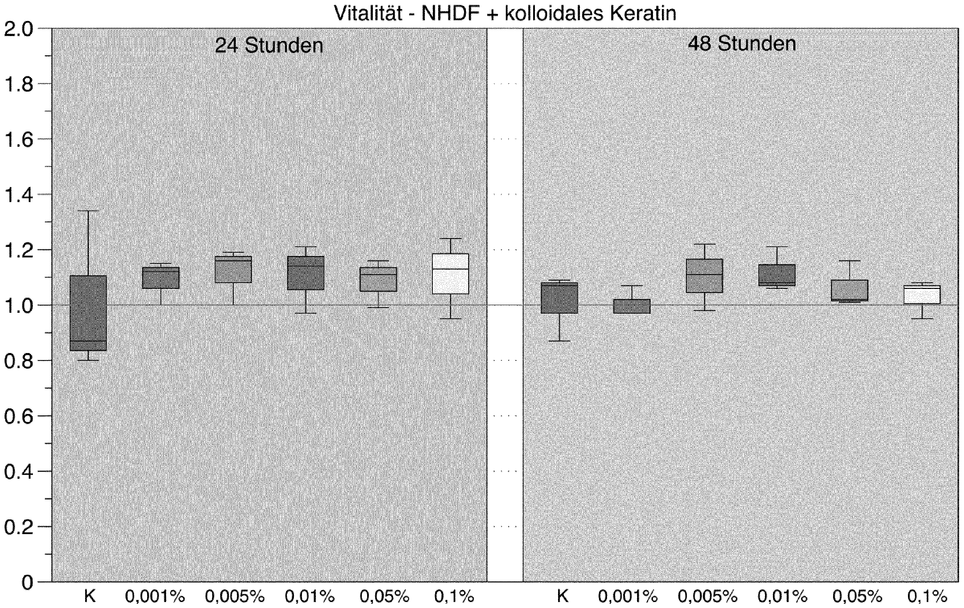

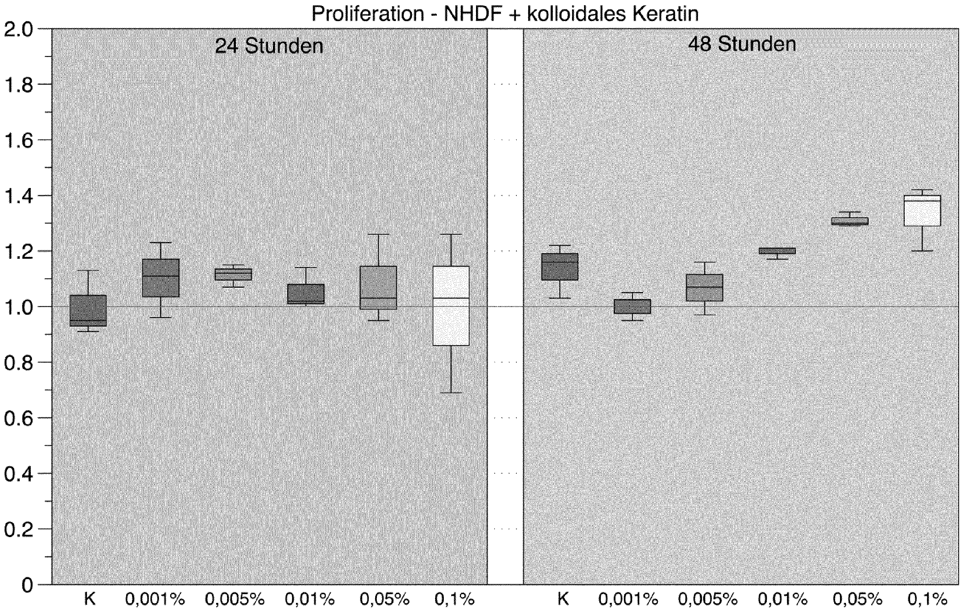

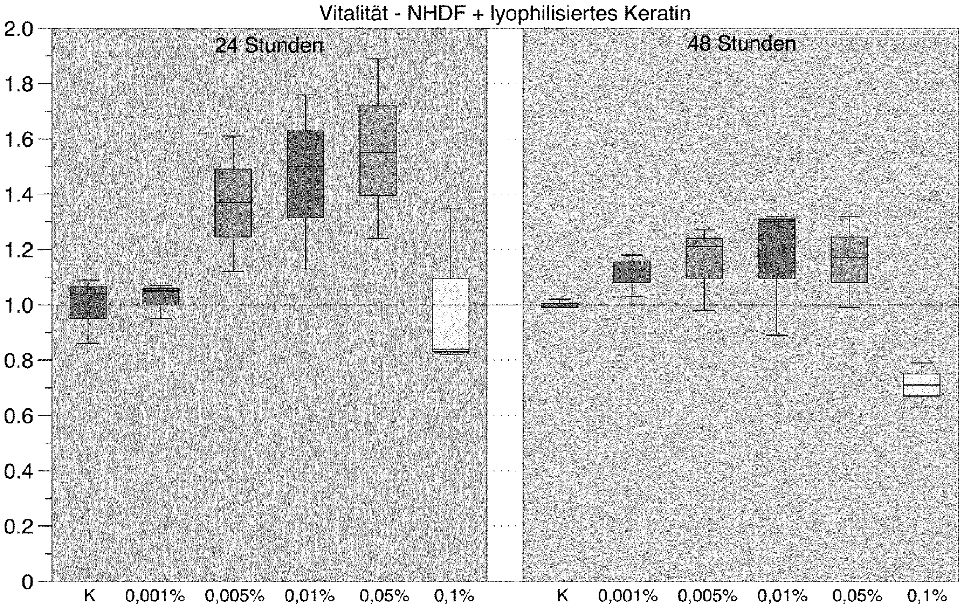

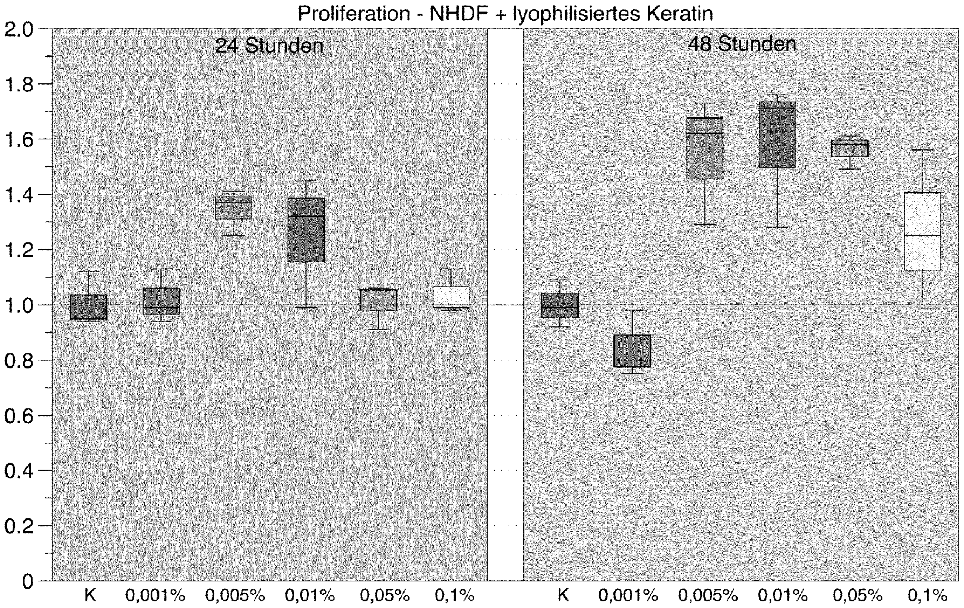

In Zytotoxizitätsuntersuchungen wurde anhand von Standardprotokollen der Einfluss von kolloidale und lyophilisierten Keratinpartikeln auf die Vitalität und proliferative Aktivität von Keratinozyten und dermalen Fibroblasten untersucht. Hierbei fand sich bis 48 Stunden Inkubationszeit kein relevanter Effekt auf dermale Fibroblasten (Fig. 6). Bei Keratinozyten zeigte sich lediglich bei kolloidalem Keratin eine leichte, konzentrationsabhängige Reduktion der proliferativen Aktivität.

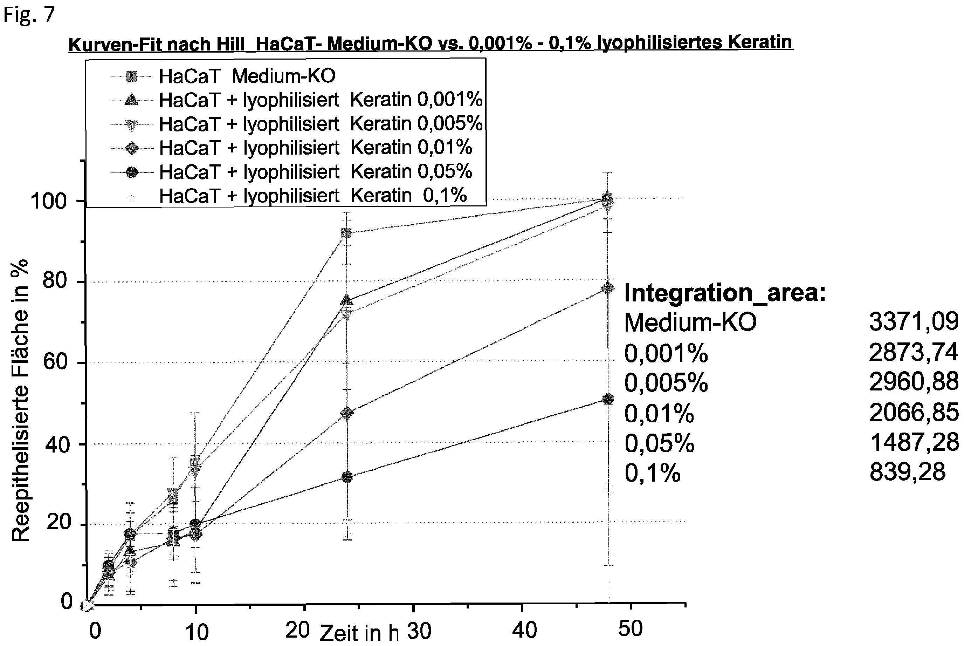

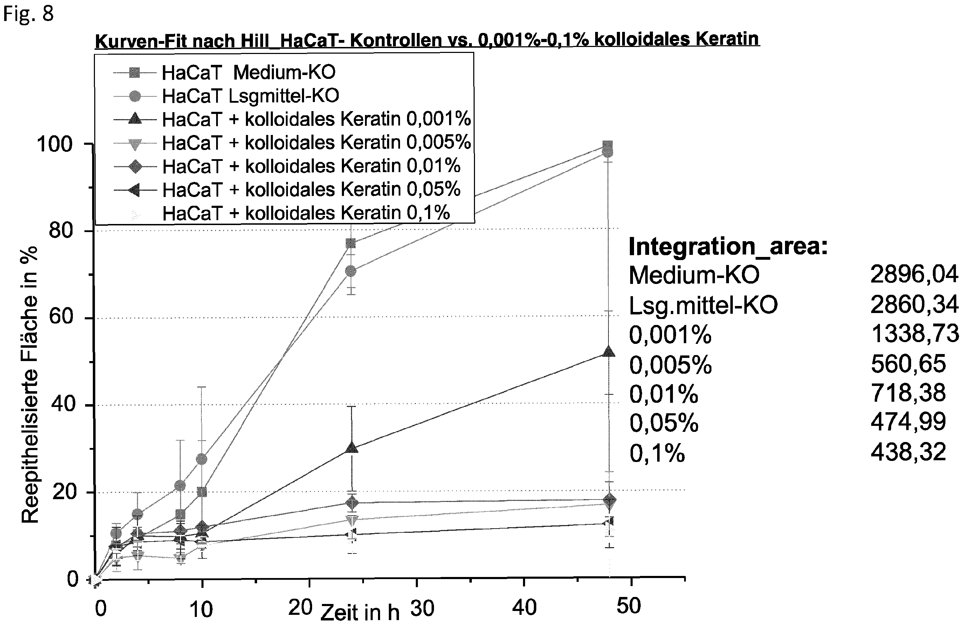

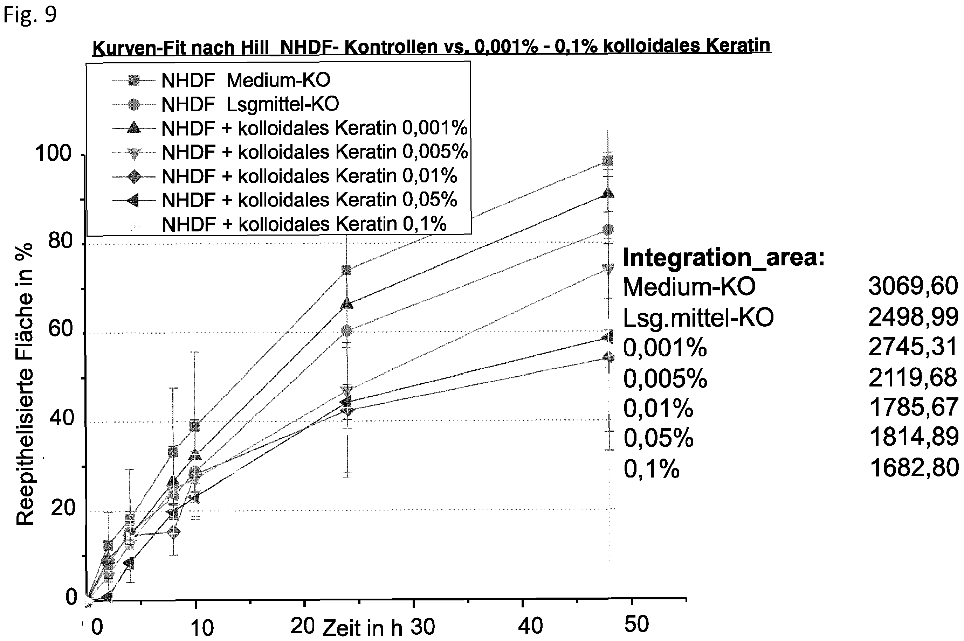

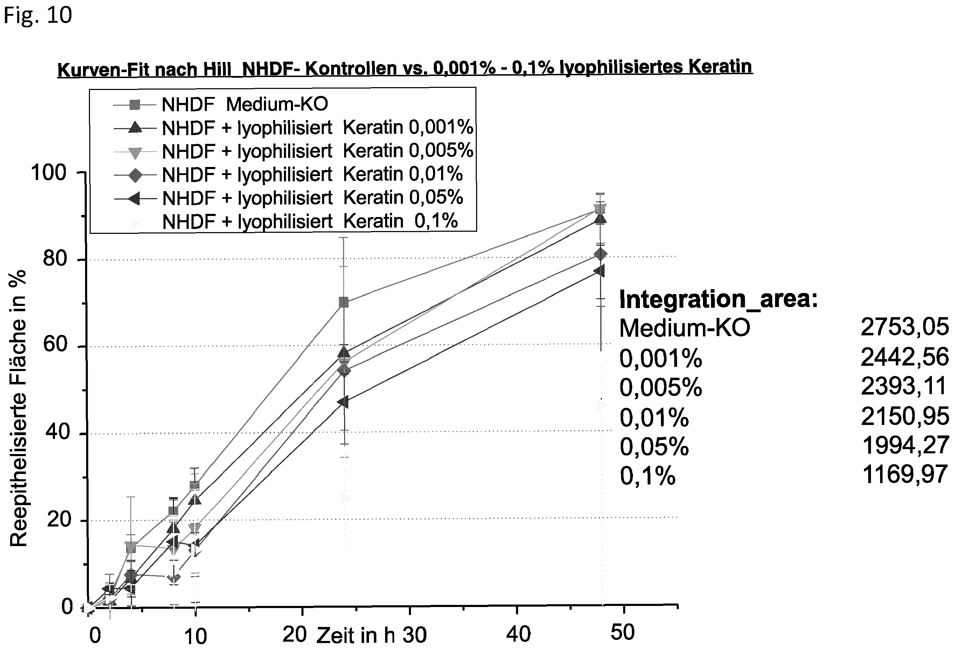

Mittels Scratch-Assay wurde der Einfluss der Keratinpartikel in kolloidaler und lyophilisierter Form auf das Migrationsverhalten von Keratinozyten (HaCaT) und dermalen Fibroblasten (NHDF) untersucht (Fig. 7 bis 10). Dabei zeigte sich eine offensichtlich mechanisch bedingte Migrationshemmung in Abhängigkeit von der Konzentration der kolloidalen und in deutlich geringerem Ausmaß der lyophilisierten Keratinpartikel. Hinweise für toxische Effekte fanden sich hingegen nicht.

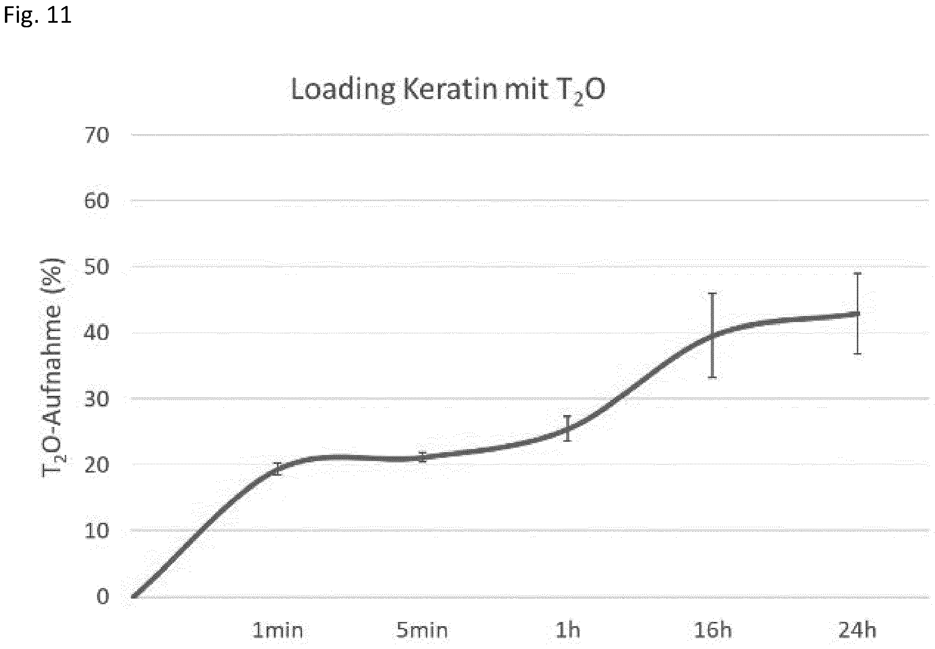

Um die Beladungsfähigkeit der Federkeratinpartikel für hydrophile bzw. hydrophobe Substanzen zu objektivieren, wurden Loading- und Deloading-Versuche durchgeführt. Zunächst wurden die Keratinpartikel in T₂O-Lösung unterschiedlich lange inkubiert und anschließend durch Zentrifugation die Menge des aufgenommenen Tritiums gemessen (Fig. 11). Im Pellet konnten nur 25-32 % des eingesetzten T₂O wiedergefunden werden. Somit zeigte sich eine unerwartet hohe Interaktion sowohl zwischen hydrophilen Substanzen und dem extrahierten Federkeratin.

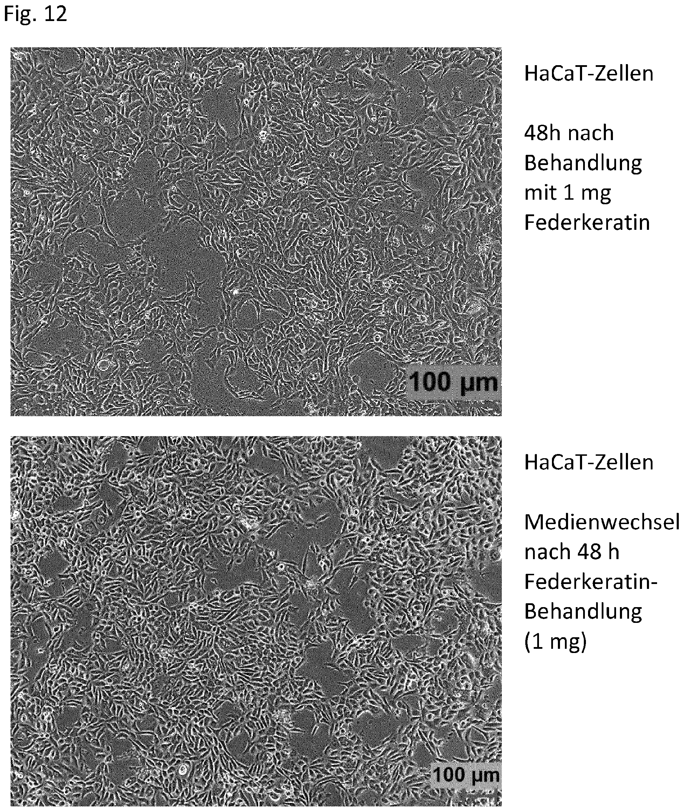

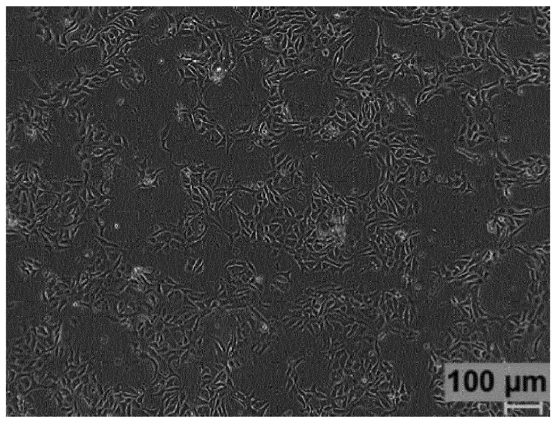

Um mögliche Interaktionen der Keratinpartikel mit vitalen Zellen zu untersuchen, wurden HaCaT-Zellen (Keratinozyten) mit fluoreszenz-markiertem Feder- und Haarkeratinpartikeln kultiviert. 48 h nach Keratinbehandlung wurde das Medium gewechselt. Die Keratinpartikel sind fluoreszensmikroskopisch dabei gut zu erkennen und zeigen eine Assoziation in der Nähe des Zellkerns, sodass eine Aufnahme der Partikel in die Zelle fluoreszenzmikroskopisch nicht auszuschließen war (Fig. 12). Deshalb wurde mittels Raman-Spektroskopie die Adhärenzzone von Keratinpartikel mit der Zellmembran untersucht, wobei keine Hinweise für intrazytoplasmatische Keratinpartikel gefunden werden konnte. Somit ist aufgrund es Zeta-Potentials und der Ladung der Phospholipide der Zellmembran von einer Adhärenz der Keratinpartikel an Zellmembranen auszugehen.

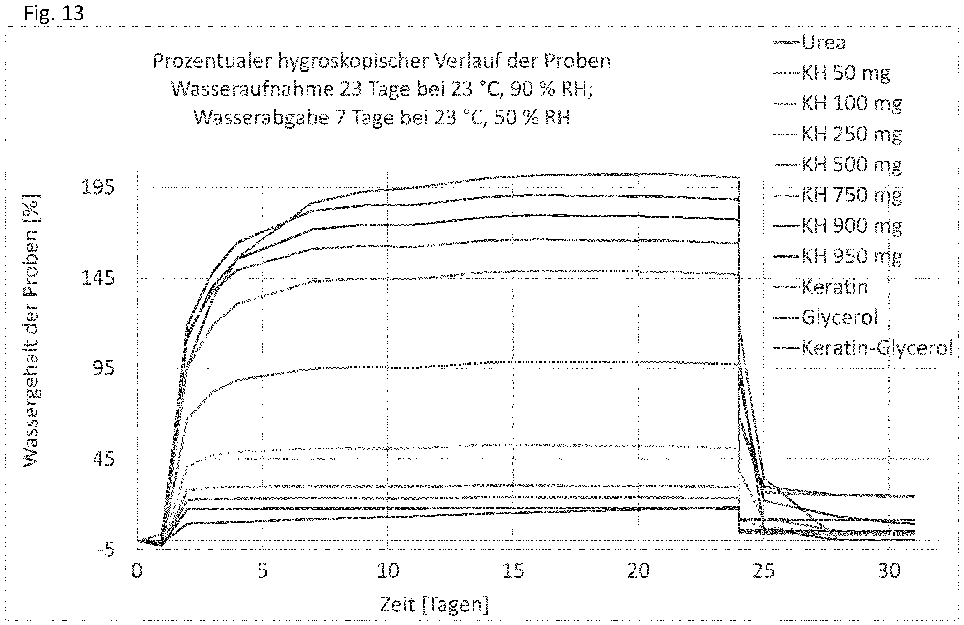

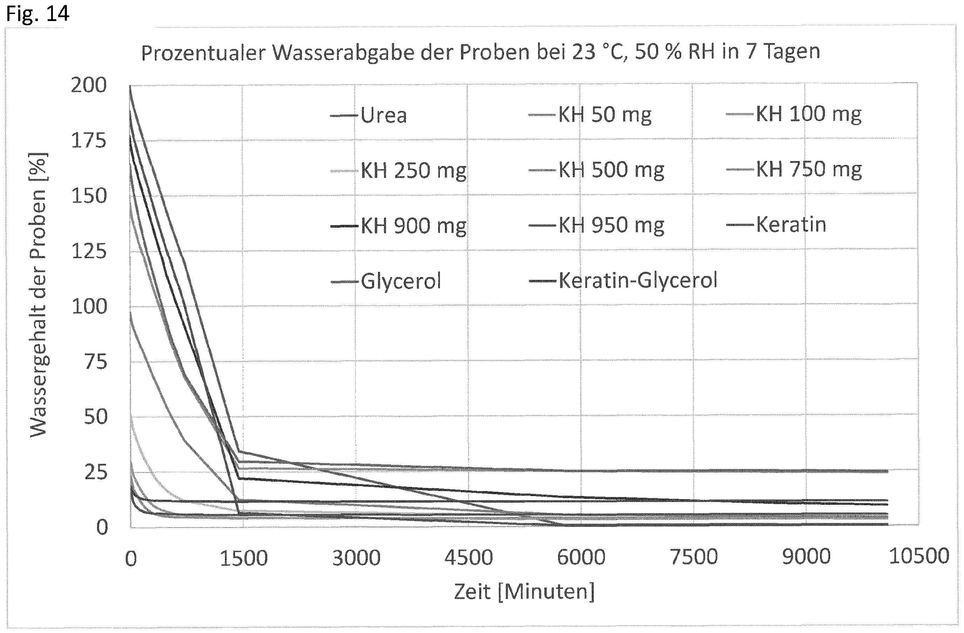

Zusätzlich wurden Hygroskopie-Versuche durchgeführt, um die Wasseraufnahmefähigkeit der Keratinpartikel, in Anlehnung an die Funktionsweise von Korneozyten zu ermitteln. Dabei zeigte sich für die Keratinpartikel nach bereits 3 Tagen eine maximale Wasseraufnahme von ca. 20%. Bei Zugabe von Harnstoff bis zu einem Verhältnis von 5% Keratin zu 95% Harnstoff wurde das Maximum immer später erreicht, im Maximalfall erst nach 15 Tagen. Hier lag die Wasseraufnahmekapazität bei 197%. (Fig. 13). Die Wasserabgabe wurde bei Raumtemperatur und einer Luftfeuchtigkeit von 50% bestimmt. Reines Keratin gab was Wasser langsam ab und enthielt selbst nach 7 Tagen noch Wasser, während für Harnstoff eine rasche Wasserabgabe von ca. 170% innerhalb der ersten 24 Stunden bis zu einer kompletten Wasserabgabe nach 4 Tagen beobachtet wurde. Für die Mischungen von Keratin und Harnstoff in den Verhältnissen 25:75 und 10:90 und 5:90 wurde selbst nach 4 Tagen noch ein Wassergehalt von 25 % beobachtet (Fig. 14).

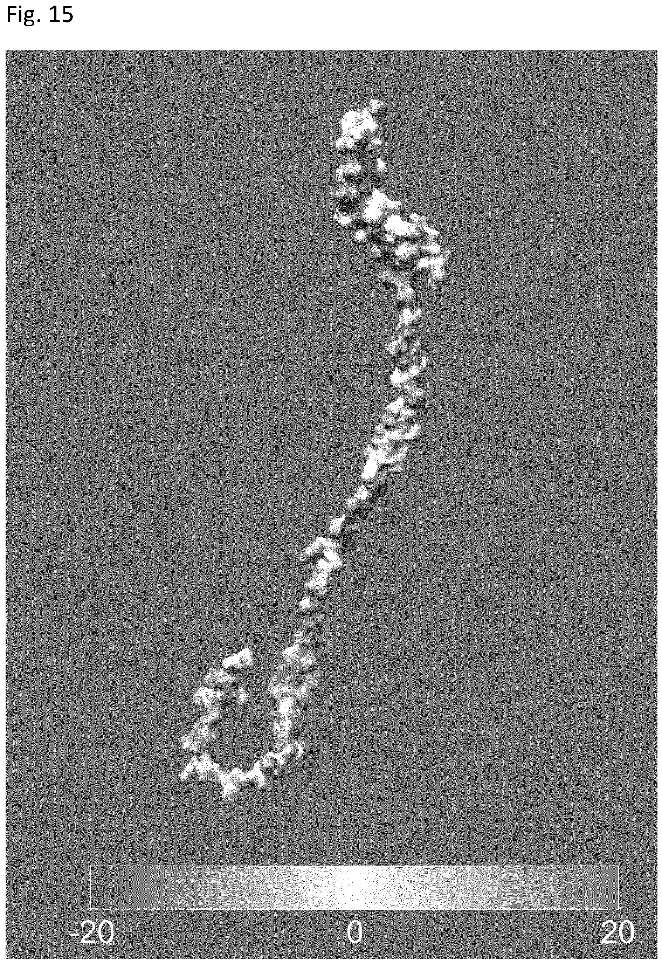

Gemäß der bekannten Keratinsequenz und der Aminosäure-Einteilung nach Dan Cojocari enthält Keratin lediglich ca. 30% hydrophobe und etwa ebenso viele hydrophile Aminosäuren, aber keine Bindungstaschen. Deshalb tritt die nachgewiesene Interaktion von Keratin mit Substanzen, die verschiedene physikochemische Eigenschaften besitzen, nicht vorhersehbar auf. Mittels der künstlichen Intelligenz (KI) AlphaFold 2 (EMBL's European Bioinformatics Institute (EMBL-EBI), Hinxton, UK) wurde deshalb die Tertiärstruktur des Keratins berechnet und die hydrophoben und hydrophilen Bereiche im Molekül identifiziert (Fig. 15). Auch hier bestätigte sich die Vermutung des Fehlens von Bindungstaschen, so dass die beobachteten Interaktionen lediglich auf der Grundlage hydrophober bzw. hydrophiler Interaktionen zwischen Aminosäurehäufungen proteinseitig mit relevanten Gruppen anderer Moleküle erklärt werden können.

## Patentansprüche

1. Verfahren zur Herstellung eines Keratinpulvers aus Federn tierischen Ursprungs, bei dem
a) eine Extraktion des Proteins beta-Keratin in einer eine chemische Denaturierung auslösenden Extraktionslösung enthaltend mindestens ein Denaturierungsagens, mindestens eine Base, mindestens ein Reduktionsmittel und mindestens eine Puffersubstanz erfolgt,
b) die Extraktionslösung aus Schritt a) einer Filtration unterzogen wird, bei der eine kolloidale Lösung von Keratinpartikeln des Proteins beta-Keratin erhalten wird,
c) die kolloidale Lösung von Keratinpartikeln aus Schritt b) per Gefriertrocknung, Sprühtrocknung, Vakuumtrocknung, Lufttrocknung, Wärmetrocknung, Infrarottrocknung und/oder Mikrowellentrocknung getrocknet wird unter Erhalt eines Keratinpulvers.

2. Verfahren nach Anspruch 1,
**dadurch gekennzeichnet, dass** die Extraktionslösung einen pH-Wert von 8 bis 13 aufweist.

3. Verfahren nach Anspruch 1 oder 2,
**dadurch gekennzeichnet, dass** das mindestens eine Denaturierungsagens ausgewählt ist aus der Gruppe bestehend aus Harnstoff, Thioharnstoff, Guanidinhydrochlorid, Natriumdodecylsulfat (SDS) und Mischungen hiervon
und/oder
die mindestens eine Base ausgewählt ist aus der Gruppe bestehend aus Natriumhydroxid, Kaliumhydroxid und Mischungen hiervon
und/oder
das mindestens eine Reduktionsmittel ausgewählt ist aus der Gruppe bestehend aus β-Mercaptoethanol, Cysteaminen, Cysteine, Glutathion, Natriumdisulfit, Natriumsulfid, Natriumhydrogensulfit, Natriumdithionit, Natriumthiosulfat, Dithiotreitol (DTT), Thioglykolsäure und ihre Salze, Thioharnstoff, Tricarboxyethylphosphan (TCEP) und andere Phosphane, Ammoniumchlorid und Mischungen hiervon
und/oder
die mindestens eine Puffersubstanz, bevorzugt ausgewählt aus der Gruppe bestehend aus Tris(hydroxymethyl)aminomethan, Natriumdodecylsulfat (SDS), Tris/Salzsäure (HCl), Ethylendiamintetraessigsäure (EDTA)/Tris, Kaliumchlorid-Natriumhydroxid (KCI-NaOH), Natriumhydrogencarbonat (NaHCO₃), Dithiotreitol (DTT)/Tris und Mischungen hiervon.

4. Verfahren nach einem der Ansprüche 1 bis 3,
**dadurch gekennzeichnet, dass** die Extraktionslösung mindestens ein Oxidationsmittel enthält, vorzugsweise ausgewählt aus der Gruppe bestehend aus Wasserstoffperoxid, Kaliumpermanganat, Natriumperborat, Peroxyessigsäure, Perameisensäure und Mischungen hiervon
und/oder
die Extraktionslösung mindestens eine Säure enthält, vorzugsweise ausgewählt aus der Gruppe bestehend aus Salpetersäure, salpetrige Säure, hypo-und hyperhalogenige Säuren und Mischungen hiervon
und/oder
die Extraktionslösung mindestens ein ionisches Agens enthält, vorzugsweise ausgewählt aus der Gruppe bestehend aus 1-Butyl-3-methyl-imidazolium (BMIM)-chlorid, 1-Butyl-3-methyl-imidazolium (BMIM)-Bromid, 1-Butyl-3-methyl-imidazolium (BMIM)-tetrafluoroborat, Amidchlorid und Mischungen hiervon.

5. Verfahren nach einem der Ansprüche 1 bis 4,
**dadurch gekennzeichnet, dass** bei der Extraktion in Schritt a) mindestens einer der folgenden Schritte erfolgt:
a) eine mechanische Zerkleinerung, insbesondere durch Zermahlen via Ultraschall, bevorzugt im Frequenzbereich von 20 bis 50 Hertz, mit einer Kugelmühle und/oder mit einer Schneidmühle, bevorzugt Ultrathurax,
b) eine thermische Denaturierung, insbesondere bei Temperaturen von 70°C bis 150°C, und/oder elektrochemische Denaturierung,
c) eine Fällung der Extraktionslösung aus Schritt a), insbesondere ausgelöst durch eine pH-Änderung, Zugabe eines Co-Solvens und/oder eines Salzes,
d) eine mikrobielle und enzymatische Extraktion über
• gram negative Bakterien ausgewählt aus der Gruppe bestehend aus *Stenotrophomonas* sp., *Chrysebacterium* sp., *Vibrio* sp. und Mischungen hiervon,
• gram positive Bakterien ausgewählt aus der Gruppe bestehend aus *Bacillus sp., Kocuria rosea* und Mischungen hiervon
• saprophytische und/oder parasitische Pilze und/oder
• Mischungen hiervon,
e) Behandlung mit Mikrowellenstrahlung, insbesondere Mikrowellenstrahlung bis zu 960 Watt und 2450 Hertz,
f) Einsatz von Stromexplosion und/oder überkritischem Wasser und/oder.
g) Kombinationen hiervon.

6. Verfahren nach einem der vorhergehenden Ansprüche,
**dadurch gekennzeichnet, dass** das Protein beta-Keratin in seiner Sekundärstruktur als β-Faltblatt vorliegt.

7. Verfahren nach einem der vorhergehenden Ansprüche,
**dadurch gekennzeichnet, dass** die Filtration über Dialyse und/oder Ultrafiltration (Cross-Flow-Filtration) erfolgt.

8. Verfahren nach einem der vorhergehenden Ansprüche,
**dadurch gekennzeichnet, dass** die Federn tierischen Ursprungs ausgewählt sind aus der Gruppe bestehend aus Federn von Hühnern, Gänsen, Puten, Enten, Truthähnen, Fasanen, Straußen, Nandus, Emus, Wachteln und Mischungen hiervon.

9. Kolloidale Lösung für die topische Applikation enthaltend Keratinpartikel des Proteins beta-Keratin und/oder deren Agglomerate aus Federn tierischen Ursprungs.

10. Kolloidale Lösung nach Anspruch 9,
**dadurch gekennzeichnet, dass** die Keratinpartikel eine Partikelgröße im Bereich von 5 bis 500 nm, bevorzugt von 70 bis 350 nm aufweisen.

11. Kolloidale Lösung nach einem der Ansprüche 9 oder 10,
**dadurch gekennzeichnet, dass** das Protein beta-Keratin in seiner Sekundärstruktur als β-Faltblatt vorliegt.

12. Kolloidale Lösung nach einem der Ansprüche 9 bis 11,
dass die Federn tierischen Ursprungs ausgewählt sind aus der Gruppe bestehend aus Federn von Hühnern, Gänsen, Puten, Enten, Truthähnen, Fasanen, Straußen, Nandus, Emus, Wachteln und Mischungen hiervon.

13. Kolloidale Lösung nach einem der Ansprüche 9 bis 12,
**dadurch gekennzeichnet, dass** zur Stabilisierung der kolloidalen Lösung Additiva enthalten sind, bevorzugt ausgewählt aus der Gruppe bestehend aus
• Proteinen, insbesondere Albumine
• Kohlenhydraten, insbesondere Saccharose, Lactose, Glucose, Fructose, Mannitol, Sorbitol und Süßstoffen wie Saccharin-Natrium, Natriumcyclamat, Aspartam, Stärke und modifizierte Stärke, Cyclodextrine und/oder Mischungen hiervon,
• Polyanionische Tenside, insbesondere Natriumdodecylsulfat, Natriumcetylstearylsulfat, Cetylstearylalkohol (emulgierend), Natriumdioctylsulfosuccinat und/oder Mischungen hiervon,
• Nichtionische Tenside, insbesondere Fettalkohole und Sterole, Sortitanfettsäureester, Polyoxyethylen-Sorbitanfettsäureester, Polyoxyethylen-Fettsäureglyceride, Macrogol-1000-glycerolmonofettsäureester, Polyoxyethylen-Fettsäureester, Polyoxyethylen-Fettalkoholether, Glycerolfettsäureester, Saccharosefettsäureester, Poloxamere und/oder Mischungen hiervon,
• Gelbildner, insbesondere Polyacrylate, Cellulosederivate, wie Methycellulose, Methylhydroxyproylcellulose Hydroxypropy-Icellulose, Hydroxyethylcelluloseund/oder Ethylcellulose, Carmellose-Natrium und/oder Mischungen hiervon,
• Verdickungsmittel, insbesondere Tragant, Xantham, arabisches Gummi, Guargalactomannan, Alginate, Bentonit, und/oder Mischungen hiervon,
• Filmbildnern, insbesondere Methacrylsäure-Acrylaten, Polyvidon, Polyvinylalkohol und/oder Mischungen hiervon,
• Polymeren insbesondere Macrogole, Gelatine und/oder Mischungen hiervon.

14. Keratinpulver für die topische Applikation herstellbar aus der kolloidalen Lösung nach einem der Ansprüche 9 bis 13, insbesondere nach dem Verfahren nach einem der Ansprüche 1 bis 8.

15. Keratinpulver nach Anspruch 19,
**dadurch gekennzeichnet, dass** die Keratinpartikel des Keratinpulvers eine Partikelgröße im Bereich von 1 bis 250 µm, bevorzugt von 5 bis 30 µm aufweisen.

16. Verwendung einer kolloidalen Lösung nach einem der Ansprüche 9 bis 13 oder des Keratinpulvers nach Anspruch 14 oder 15 zur Herstellung von Zubereitungen mit therapeutischer, diagnostischer, präventiver oder kosmetischer Applikation an Lebewesen, insbesondere an Menschen und/oder Tieren.

17. Verwendung nach Anspruch 16,
**dadurch gekennzeichnet, dass** die kolloidale Lösung mindestens einen kleinmolekularen und/oder biotechnologischen Wirkstoff enthält.

18. Verwendung nach Anspruch 16 oder 17,
**dadurch gekennzeichnet, dass** der Wirkstoff ausgewählt ist aus der Gruppe bestehend aus Glucocorticoiden, Calcineurininhibitoren, Januskinase-Inhibitoren, Antibiotika, und/oder selbst ein Protein, Fraktionsprotein, Peptid, Enzym, Antikörper, Antikörperfragment, RNA- und/oder DNA-Molekül, und Mischungen hiervon oder ein Molekül, das ein Target für einen dieser Wirkstoffe darstellt.

19. Verwendung nach einem der Ansprüche 16 bis 18,
**dadurch gekennzeichnet, dass** die kolloidale Lösung kosmetische Wirk-und Hilfsstoffe enthält, bevorzugt ausgewählt aus der Gruppe bestehend aus Aminosäuren, Harnstoff, Glycerol, Hyaluronsäure, Zuckern- und zuckerähnlichen Derivaten, Extrakten oder Wachsen aus Pflanzen oder von Tieren stammenden Teilen und Mischungen hiervon.

20. Verwendung nach einem der Ansprüche 16 bis 19,
**dadurch gekennzeichnet, dass** spezifische und/oder unspezifische Verbindungen mit Wirk- und/oder Hilfsstoffen durch kovalente und/oder nicht-kovalente Bindungen, bevorzugt über ausgewählte Gruppen bestehend aus Carbonsäuregruppe, Aminogruppen, Thiolgruppen und/oder Hydroxylgruppen, gebildet werden.

21. Verwendung nach einem der Ansprüche 16 bis 20,
**dadurch gekennzeichnet, dass** die kolloidale Lösung mit/ohne Wirk-und/oder Hilfsstoffe diese als liposomale Systeme in uni- oder multilamellare Vesikel unterschiedlicher oder einheitlicher Größe eingebaut ist durch Zusatz von amphiphilen Molekülen, bevorzugt ausgewählt aus der Gruppe bestehend aus
• Phospholipiden, bevorzugt Lecithin, DODAB, DPPC, DSPC, DSTAP und/oder Mischungen hiervon,
• kationischen Lipiden, bevorzugt ALC-0315,
• PEGylierte Lipide, bevorzugt ALC-0159
• Prostaglandine und -modifikationen, bevorzugt PGE1, PGD2, PGE2, 15-keto PGE1 und/oder Mischungen hiervon,
• Ceramiden, bevorzugt ausgewählt aus der Gruppe bestehend aus Ceramiden der Kopfgruppen NS, NH, NP, NDS, AS, AH, ADS, AP der Kettenlängen C₁₀ bis C₂₆ und/oder Mischungen hiervon,
• Ceramiden, bevorzugt ausgewählt aus der Gruppe bestehend aus Ceramiden der Kopfgruppen EOS, EOH, EOP der Kettenlängen C₁₀ bis C₃₂ und/oder Mischungen hiervon,
• Cholesterol, Cholesterolderivaten und/oder Mischungen hiervon,
• Fettsäuren, bevorzugt ausgewählt aus der Gruppe bestehend aus Fettsäuren der Kettenlängen C₁₀ bis C₃₂ und/oder Mischungen hiervon.

22. Verwendung nach Anspruch 21,
**dadurch gekennzeichnet, dass** die Applikation an Lebewesen, bevorzugt am Menschen, die Haut, Schleimhaut (inkl. Konjunktiven) oder den Hautanhangsgebilden (inkl. Nägeln und Haare) betrifft, insbesondere zur Substitution der Barrierefunktion bzw. von Bestandteilen der epidermalen Barriere.

23. Verwendung nach Anspruch 21 oder 22 zur Herstellung von
• flüssigen Grundlagen, insbesondere Lösungen, Emulsionen, Suspensionen und/oder Kolloiden,
• halbfesten Grundlagen, insbesondere Suspensionssalben, Salben, Cremes, Gele, Pasten, Kolloiden und/oder Suppositorien,
• fester Grundlagen, insbesondere Pulver, Tabletten, Granulate, Pellets, Kapseln und/oder Inserts.

24. Verwendung nach einem der Ansprüche 21 bis 23 zur Herstellung von
• Nahrungsmitteln und Nahrungsergänzungsmitteln,
• Tierfutter,
• Dünge- und/oder Pflanzenschutzmitteln für Pflanzen und/oder Böden,
• technischen Hilfsstoffen z.B. in/als Filtersystem, Klebstoff- und/oder Haftvermittler, Konsistenzgeber, Füllstoff, Absorber für hydrophile oder lipophile Stoffe, geladene oder ungeladene Stoffe,
• Verpackungsmaterialien und/oder Bedarfsgegenständen,
• Textilien und/oder Fasern mit/ohne Funktion, insbesondere wasserabweisend, atmungsaktiv,
• Medizinprodukten, insbesondere Pflaster, Wundauflagen, Tamponaden, wund- und/oder hautversorgende Kleidung.
